(19) Europäisches Patentamt
European Patent Office
Office européen des brevets

(11) EP 3 125 770 B1

(12) **EUROPEAN PATENT SPECIFICATION**

(45) Date of publication and mention
of the grant of the patent:
**06.06.2018 Bulletin 2018/23**

(21) Application number: **15716859.2**

(22) Date of filing: **01.04.2015**

(51) Int Cl.:
***A61B 8/08*** *(2006.01)*    ***G01S 7/52*** *(2006.01)*

(86) International application number:
**PCT/GB2015/051024**

(87) International publication number:
**WO 2015/150810 (08.10.2015 Gazette 2015/40)**

(54) **ULTRASONIC CONTRAST AGENT DETECTION AND IMAGING**

ULTRASCHALLKONTRASTMITTELDETEKTION UND -BILDGEBUNG

DÉTECTION ET IMAGERIE D'AGENT DE CONTRASTE ULTRASONORE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.04.2014 GB 201405848**

(43) Date of publication of application:
**08.02.2017 Bulletin 2017/06**

(73) Proprietor: **Sintef TTO AS
7465 Trondheim (NO)**

(72) Inventors:
• **HANSEN, Rune
7465 Trondheim (NO)**
• **BERG, Sigrid
7465 Trondheim (NO)**

(74) Representative: **Gardiner, Stephen Robin
Dehns
St Bride's House
10 Salisbury Square
London EC4Y 8JD (GB)**

(56) References cited:
WO-A1-2006/021400        WO-A1-2010/121265
JP-A- 2002 209 895        US-A1- 2002 028 994
US-A1- 2004 236 222        US-A1- 2004 267 129

• MAYER S ET AL: "MYOCARDIAL CONTRAST
AGENTS: RECENT ADVANCES AND FUTURE
DIRECTIONS", PROGRESS IN
CARDIOVASCULAR DISEASES, SAUNDERS,
PHILADELPHIA, PA, US, vol. 44, no. 1, 1 July 2001
(2001-07-01), pages 33-44, XP001051429, ISSN:
0033-0620, DOI: 10.1053/PCAD.2001.26438

**Description**

The invention relates to methods and apparatus for detecting and imaging ultrasound contrast agents. In some preferred forms, the invention relates to methods for ultrasonic detection and imaging of contrast agents located in soft tissue or tissue fluids.

Ultrasound contrast agents are usually made as solutions of gas microbubbles, with a diameter in the range of 1-5 $\mu$m, that can be intravenously injected to increase the scattering from blood which is weak compared to the scattering from soft tissues. Scattering from the microbubble is resonant through an interaction between the co-oscillating fluid mass around the bubble and the bubble compression elasticity. The microbubble has high compliance relative to the surrounding blood and when driven by ultrasound pulses with frequencies below or in the vicinity of its resonance frequency, large radius excursions are achieved due to mainly shear deformation and limited volume compression of the surrounding blood. Conventional microbubbles have thin (typically in the range of 1-20 nm) and flexible shells made from either lipid or albumin that stabilize the gas core. The local nonlinear scattering from conventional ultrasound contrast agents is typically much larger than from soft tissues. The nonlinearly scattered signal from these conventional microbubbles is used to enhance the contrast agent signal above the tissue signal in methods generally referred to as nonlinear contrast harmonic imaging.

Two signal power ratios are of significant importance for the quality of performance of a contrast imaging system. Firstly, the Contrast signal to Tissue signal Ratio (CTR) gives the ratio of the signal power scattered from the contrast agent in a region to the signal power scattered from the tissue in that region. This ratio is often referred to as specificity. Secondly, the Contrast signal to Noise Ratio (CNR) gives the ratio of the signal power scattered from the contrast agent in a region to the noise power in that region. This ratio is often referred to as sensitivity.

The CNR determines the maximum depth for imaging the contrast agent. In general, it is desirable that the CNR is adequate for detection and imaging of individual microbubbles. The CTR describes the enhancement of the contrast agent signal above the tissue signal and hence the capability of differentiating contrast signal from tissue signal. High values of both these signal ratios are desired for robust imaging of the contrast agent.

Because the shell of a conventional bubble is thin and flexible, nonlinear harmonic components are scattered from the microbubble also at low amplitude incident pressure pulses unless the imaging frequency is well above the bubble resonance frequency. Wave propagation at low transmit pressures will be close to linear and by utilizing the nonlinear harmonic components scattered from conventional microbubble at these low transmit pressures, adequate contrast agent specificity can often be obtained. However, the commercially available thin-shelled microbubbles have limitations, for example with respect to fragility, blood circulation time, multi-functionality and capacity for drug loading and drug delivery. To overcome important limitations found with conventional microbubble-based contrast agents, several research groups are therefore working with development of novel microbubbles. Several of these novel microbubbles under development have an encapsulating shell based on biocompatible polymers and the shell thickness is typically increased compared with conventional thin-shelled microbubbles such as SonoVue®.

US 2002/0028994 describes a microbubble imaging technique to cancel steady state echoes from tissue, but not non-steady state echoes. Excitations are used to change the size and/or collapse of the microbubbles. US 2004/0236222 describes an imaging technique using two or more frequency components positioned closely in time so as to generate an intermodulation signal from the microbubble which is useful in the vicinity of resonance and on thin-shelled microbubbles. US 2004/0267129 describes an imaging technique using dual-frequency pulse complexes consisting of overlapped low- and high-frequency pulses to image different compression states of microbubbles.

The new method described here is especially suitable for imaging of microbubbles having a reduced fragility compared to conventional microbubbles such as SonoVue® and for imaging of conventional microbubbles well above their resonance frequencies.

According to a first aspect, the invention provides a method for detection and imaging of ultrasound contrast agent in a region, where: at least two ultrasound pulses are transmitted consecutively toward said region along a given radial image line; at least one of said transmitted pulses induces a physical movement and/or at least transiently alters the acoustic scattering properties of the contrast agent; the received signals from said at least two transmitted pulses are used as imaging signals to detect and image the contrast agent; and the imaging signals are for each depth along said radial image line combined such that the signal components from the contrast agent are, at least to some degree, preserved for detection and imaging of the contrast agent while other signal components are suppressed; and wherein one or more of the transmitted pulses has a mechanical index greater than 0.3; and/or the imaging frequency is at least three times higher than the resonance frequency of the contrast agent.

The method is based on transmitting at least two ultrasound pulses along each radial image line of an ultrasound image, where the purpose is to introduce a physical movement and/or a change in ultrasound scattering properties for the contrast agent.

The image reconstruction is based on combining the received echoes resulting from at least two transmit pulses for suppression of tissue signal and detection and imaging of contrast agent signal.

The at least one transmitted pulse that induces movement or a change in scattering properties may be the first imaging pulse itself or it may be a subsequent pulse intermediate between the two imaging pulses. Where more than two imaging pulses are used, intermediate pulses may be used between each adjacent pair of imaging pulses. The use of separate intermediate pulses allows greater versatility by allowing the imaging pulses to be designed for imaging and the intermediate pulses to be designed for inducing movement or altering scattering properties as desired. As echoes from each successive imaging pulse are received, the ultrasound contrast agent that caused the echo will have been affected by all preceding pulses (both imaging and where applicable intermediate) and thus successive echoes will reveal increasingly greater effects of the preceding pulses, e.g. increasing movement of the contrast agent.

The method may be applied to any substance where contrast agent is used to distinguish from other materials. However, one of the main areas of applicability is in medical imaging and thus in some preferred embodiments the region at which the ultrasound is directed is a region of soft tissue and the imaging signals are combined so as to suppress tissue signal components while preserving, at least to some degree, those of the contrast agent.

The method can be applied along a single transmit direction if desired. However, in many applications, it will be advantageous to perform several measurements along adjacent lines where the adjacent lines may be parallel. In some preferred embodiments, the ultrasound transmit direction is scanned in steps, each step corresponding to a radial image line and for each radial image line a group of pulses is transmitted; the group of pulses comprises at least two imaging pulses, each imaging pulse in the group having a pulse number; the back-scattered signal is sampled as a function of depth for each imaging pulse and the samples for each depth are combined linearly along the pulse number coordinate; the linear combination for each depth being selected so that the received signal components from the contrast agent are, at least to some extent, preserved while other components are heavily suppressed; and so that the output of the linear combination is used to form the image of the contrast agent for each depth along said radial image line.

Preferably in said transmitted pulses the amplitude may be separately adjusted. Preferably in said transmitted pulses the center frequency may be separately adjusted. Preferably in said transmitted pulses the number of oscillations may be separately adjusted. Most preferably these adjustments are made to the intermediate (non-imaging) pulses.

Preferably between said transmitted pulses the pulse repetition time may be separately adjusted. In particular, as the intermediate (non-imaging) pulses are not used for image construction, their timing relation is not critical and therefore may be modified or selected for other purposes, e.g. to maximize a desired interaction with the contrast agent.

Preferably the transmitted ultrasound pulses comprise at least two transmit imaging pulses. More than two imaging pulses may of course be used. Indeed a train of imaging pulses may be used. The imaging pulses may be identical. Identical imaging pulses can be expected to have the same effect on the stationary parts of the imaged region, e.g. those without contrast agent present.

Preferably one or more intermediate pulses are transmitted in between said at least two imaging pulses. Where a train of pulses is used, intermediate pulses may be used between any adjacent pair of imaging pulses, or indeed may be provided between several such pair or all such pairs of imaging pulses.

Preferably one or more of the transmit pulses has a frequency and/or number of oscillations and/or amplitudes suitable for inducing movement in the contrast agent that can be detected by linearly combining the received signals of the at least two transmitted imaging pulses. As described above, some of the transmitted pulses (especially the intermediate pulses if present) may be specifically shaped so as to maximize the desired interaction with the contrast agent. For example, frequency, amplitude and/or number of oscillations may be adjusted to increase the absorption and/or scattering from incident pulses for the contrast agent, e.g. for inducing movement, or these parameters may be selected so as to maximize the change in scattering properties or to induce a particular type of change in the scattering properties of the contrast agent.

One area of particular application for the invention is for microbubble contrast agents with thicker shells. One benefit of the thicker shell that is recognised here is that more powerful pulses may be directed at the microbubbles without risk of destruction. Therefore one or more of the transmitted pulses may have a mechanical index greater than 0.3 and more preferably greater than 0.5. The contrast agent may comprise microbubbles with a shell having a thickness greater than 40 nm.

One or more of the intermediate transmit pulses may have a longer duration than the first transmit imaging pulse. The longer duration permits a larger energy transfer from the pulse to the contrast agent.

In some embodiments one or more of the transmit pulses has a frequency and/or number of oscillations and/or amplitude suitable for inducing a change in the acoustic scattering properties of the contrast agent.

In some embodiments the contrast agent comprises microbubbles and wherein one or more of the transmit pulses has a frequency and/or number of oscillations and/or amplitude suitable for causing cracks or other physical changes in the shells of said microbubbles.

Another advantage of the methods described here is for imaging contrast agents significantly above their resonance frequency. Ordinarily the imaging frequency is selected so as to resonate with the contrast agent for improved imaging. However, in the methods described here, interaction with the contrast agent can be achieved via other pulses in the pulse train. Therefore the imaging frequency may be higher than the resonance frequency of the contrast agent, in some embodiments preferably at least three times higher than the resonance frequency of the contrast agent.

In some preferred embodiments, the contrast agent is microbubbles and the microbubbles are thixotropic. Thixotropic microbubbles can have their scattering properties significantly altered by application of the right pulses and are therefore of particular use in the methods described here.

In some preferred embodiments the transmitted pulses are unfocussed and cover a large field of view. Various transmit angles can be used for the unfocussed transmitted imaging pulses, and the received signals from the unfocussed transmitted imaging pulses are collected on transducer channels in parallel, beam-formed synthetically and combined to suppress tissue signal components and thereby to image the ultrasound contrast agent. In some particularly preferred embodiments the unfocussed pulses are plane wave pulses.

In some embodiments, to suppress tissue signal components due to physiologic motion or transducer motion, motion filters driven by recorded data (received echoes), model-based algorithms or by a combined approach using information from both recorded data and models may be applied.

According to a second aspect, the invention provides an apparatus for imaging an ultrasound contrast agent, comprising: a transmitter arranged to transmit at least a first imaging pulse and a second imaging pulse along a given radial image line, wherein at least one pulse transmitted prior to the second imaging pulse is capable of inducing movement in the ultrasound contrast agent or is capable of altering the acoustic scattering properties of the ultrasound contrast agent; a receiver arranged to receive echoes from the imaging pulses and generate imaging signals; a processor arranged to combine imaging signals to suppress tissue signal components for each depth along said radial image line and thereby to image the ultrasound contrast agent and wherein one or more of the transmit pulses has a mechanical index greater than 0.3; and/or the imaging frequency is at least three times higher than the resonance frequency of the contrast agent.

All of the preferred features described above in relation to the methods apply equally to the apparatus which may be suitably adapted to transmit and receive pulses and to process received signal information to accomplish any of those methods. It will be appreciated that the transmitter and receiver may be separate physical entities or they may be embodied in the same transducer, i.e. as a transceiver.

Certain preferred embodiments of the invention will now be described, by way of example only, and with reference to the accompanying drawings in which:

Figure 1 illustrates a set of echoes from consecutive transmit pulses along a given radial image line as a function of fast time (depth) and slow time (pulse number coordinate).

Figure 2 shows an example where two identical pulses are consecutively transmitted along a radial image line with a pulse repetition time (PRT) of $PRT_1$.

Figure 3 shows the gain factor $G(\omega) = 2 \sin \dfrac{\omega \tau}{2}$ as a function of delay (in nanoseconds) for an imaging frequency of 12 MHz (301) and for an imaging frequency of 6 MHz (302).

Figure 4 shows a first imaging signal 401 and a second imaging signal 402 where the only difference between the two signals is a time delay of 5 ns. These two signals have a center frequency of 12 MHz. The curve 403 is obtained as the difference between 401 and 402.

Figure 5 shows a first signal 501 and a second signal 502. These two signals have a center frequency of 12 MHz. The difference between the two signals 501 and 502 illustrates a potential change in the scattering properties from a given microbubble, for example introduced due to a thixotropic behavior of the shell encapsulating the gas core of the microbubble. The curve 503 is obtained as the difference between 501 and 502.

Figure 6 shows an example where four pulses are consecutively transmitted along a radial image line. The first and the last transmitted pulses are used for image reconstruction and are identical. The two intermediate transmit pulses have center frequencies, fractional bandwidths and amplitudes that differ from the transmitted imaging pulses. The pulse repetition time (PRT) for the imaging transmit pulses is $PRT_1$, for the intermediate transmit pulses the pulse repetition time can vary ($PRT_2 \neq PRT_3$) and may be different from $PRT_1$.

Embodiments of the invention will now be described in more detail.

For non-destructive imaging of conventional thin-shelled microbubbles, methods generally referred to as nonlinear contrast harmonic imaging are usually applied. At low transmit pressures, the wave propagation in soft tissue will be close to linear and the conventional thin-shelled microbubbles will, especially when imaged below or around their resonance frequencies, typically give a nonlinear back-scattering that can be utilized to enhance the contrast agent signal above the tissue signal. However, for microbubbles that have thicker and/or less flexible shells or for imaging well above the microbubble resonance frequency, higher incident pressure amplitudes are required to produce significant nonlinear back-scattering from the contrast agent.

In the linear regime, acoustic wave propagation is governed by the linear wave equation with a linear propagation velocity defined as

$$c_0 = \frac{1}{\sqrt{\rho\kappa}}$$

where $\rho$ is the mass density and $\kappa$ is the linear compressibility of the propagation medium. Soft tissue has a nonlinear elasticity where the instantaneous compressibility varies with the acoustic pressure $p$, resulting in the fact that the tissue gets stiffer during volume compression and softer during volume expansion. The propagation velocity $c$ will therefore be pressure dependent

$$c(p) = c_0\sqrt{1 + 2\beta_n\kappa p - 2\beta_n^2(\kappa p)^2} \approx c_0(1 + \beta_n\kappa p)$$

where the approximation is valid when $\kappa p << 1$ which is the case for medical ultrasound imaging and $\beta_n$ is a nonlinearity parameter. The resulting propagation distortion of an ultrasound imaging transmit pulse produces harmonic tissue components with signal characteristics similar to nonlinear back-scattering from contrast agents. The amplitude of the harmonic tissue components will increase with increasing transmit pressure amplitude and will limit the specificity that can be obtained with contrast agent detection schemes based on nonlinear harmonic imaging. Harmonic tissue components are utilized for suppression of acoustic noise in soft tissue imaging.

Radiation forces are produced by the absorption and/or reflection of acoustic energy causing momentum transfer from the ultrasound wave to the medium. The primary radiation force will act in the direction of the wave propagation. The radiation force $F$, the intensity $I$ of the ultrasound wave and the mechanical index $MI$ is given by

$$\langle F \rangle = \frac{\sigma_e \langle I \rangle}{c} \quad , \quad I = \frac{p^2}{\rho c} \quad , \quad MI = \frac{P_-}{\sqrt{f}}$$

where the brackets indicate time-averaging, $\sigma_e$ is the extinction cross section (the sum of absorption and scattering), $P_-$ is the amplitude of the negative pressure in MPa and $f$ is the frequency in MHz. For imaging of conventional thin-shelled microbubbles, it is common to use a low mechanical index in the range 0.03 - 0.2 to avoid bubble destruction and to obtain adequate specificity with applied pulsing schemes based on the detection of nonlinear harmonic components. With less fragile microbubbles currently being developed and for imaging of conventional microbubbles well above their resonance frequency, one can potentially use a mechanical index, and hence an acoustic pressure, that is significantly higher without causing significant bubble disruption. If for example the acoustic transmit pressure is increased by a factor of 10, the resulting wave intensity will be increased by a factor of 100 meaning that the resulting radiation force that potentially may be obtained on the microbubbles is significantly increased.

In ultrasound imaging, the depth time coordinate reflects the depth of the scatterers and is often denoted "fast time" whereas the pulse number coordinate samples slower variations and is often denoted "slow time". Variations in the slow time (or along the pulse number coordinate) will occur due to any of: 1) movement between the ultrasound probe and the scatterers due to physiologic motion and movement of the ultrasound transducer, 2) due to movement between the scatterers and the ultrasound probe induced by the transmitted ultrasound pulses or 3) due to changes in scattering properties of the scatterers induced by the transmitted ultrasound pulses. Therefore at least the preferred embodiments of the invention aim to induce variations (i.e. movement and/or change in scattering properties) in the slow time for detection and imaging of ultrasound contrast agents.

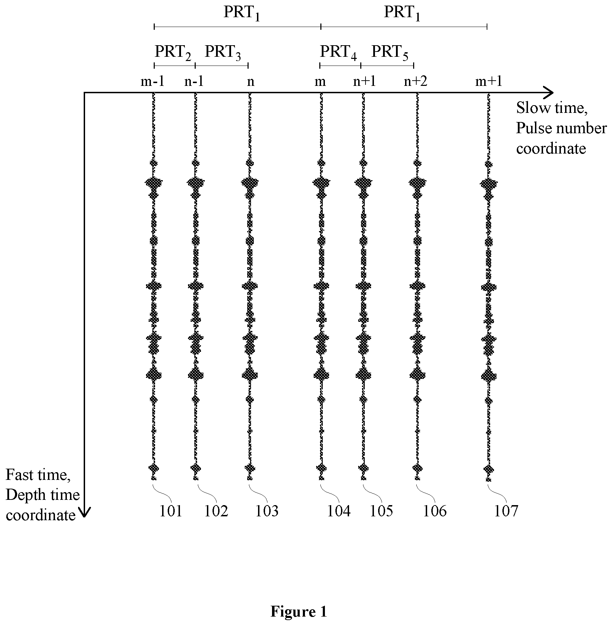

Figure 1 illustrates seven echoes (101-107) resulting from seven transmit pulses along a given radial image line. In this schematic illustration, three of the transmitted pulses are used for image reconstruction and are hence termed transmit imaging pulses while the resulting received signals (101, 104 and 107) are termed imaging signals. The pulse repetition time for the transmitted imaging pulses ($PRT_1$) is typically chosen to be a little longer than the time needed to receive the scattered signals from the deepest imaging depths. Between transmit imaging pulses, intermediate transmit pulses used to enhance movement and/or to induce change in scattering properties of the ultrasound contrast agent can be applied. For example, a total of four intermediate transmit pulses are used in this illustration but the resulting echoes (102, 103, 105 and 106) are not received and stored for image reconstruction. The pulse repetition time for the intermediate transmit pulses ($PRT_2, PRT_3, PRT_4$ and $PRT_5$) may thus be different than for the imaging transmit pulses and are not limited by the time required to receive echoes from a certain depth. As shown in Figure 1, the transmit imaging pulses and the received imaging signals are numbered using the sequence m-1, m, m+1, while the intermediate transmit pulses and intermediate echoes are numbered using the sequence n-1, n, n+1, n+2.

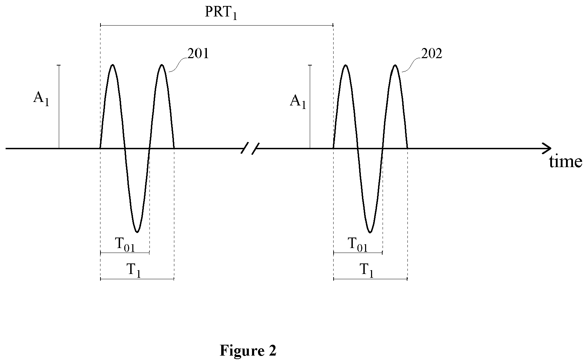

Figure 2 depicts a first transmit pulse 201 and a second transmit pulse 202 that are transmitted with a pulse repetition time (PRT) of $PRT_1$ along the same radial image line. The two transmit pulses shown in Figure 1 have the same time period $T_{01}$ and hence the same centre frequency, the same pulse duration $T_1$ and hence the same bandwidth and the same amplitude $A_1$. In its simplest form, the new detection method is performed by transmitting a first imaging pulse along a given radial image line, receiving the resulting echoes from this first transmit imaging pulse, transmitting a second identical imaging pulse along the same radial image line, receiving the resulting echoes from this second transmit imaging pulse, and finally performing a simple subtraction of the two resulting imaging echoes. For adequately stationary objects, the tissue signals will be heavily suppressed in the simple subtraction process.

Radiation forces from the two transmit pulses will induce a displacement with a corresponding delay $\tau$ between the first echo $x_1$ and the second echo $x_2$ received from a given microbubble. Assuming that the only difference between the first and the second echo received from the bubble is a delay $\tau$ due to an induced displacement, the resulting detection signal $s$ may be written as

$$s(t) = x_1(t) - x_2(t) = x(t) - x(t - \tau).$$

For adequately narrow-band signals, one can derive the following by doing a Fourier Transform and using the relationship between trigonometric functions and exponential functions

$$S(\omega) = X(\omega) - X(\omega)e^{-i\omega\tau}$$

$$S(\omega) = X(\omega)e^{-\frac{i\omega\tau}{2}}\left[e^{\frac{i\omega\tau}{2}} - e^{-\frac{i\omega\tau}{2}}\right]$$

$$S(\omega) = X(\omega)e^{-\frac{i\omega\tau}{2}}i2\sin\frac{\omega\tau}{2}$$

$$S(\omega) = X(\omega)P(\omega)G(\omega)$$

where $i$ is the imaginary unit, $\omega$ is the angular frequency of the imaging pulse, $P(\omega)$ is a phase factor defined as

$$P(\omega) = ie^{-\frac{i\omega\tau}{2}}$$

and $G'(\omega)$ is a gain factor defined as

$$G(\omega) = 2\sin\frac{\omega\tau}{2}.$$

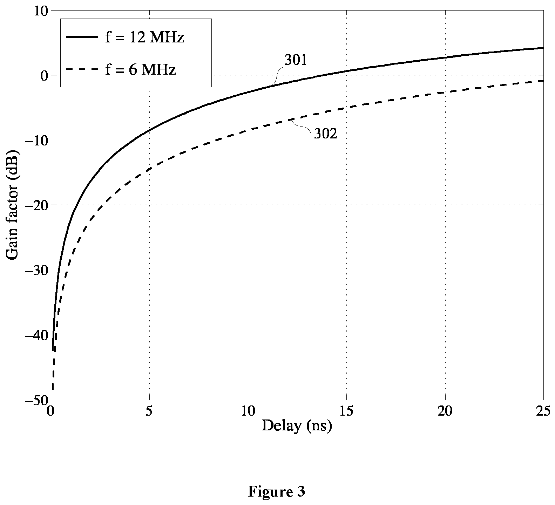

Figure 3 displays this gain factor as a function of relative delay (in nanoseconds) between the first and second echo received from a microbubble. The gain factor is mostly negative indicating a reduction in signal amplitude for the detection signal $s$ relative to the received bubble echo $x$. However, for an imaging frequency of 12 MHz the gain curve 301 is positive for delays exceeding 14 ns indicating that the detection signal then is stronger than a received bubble echo. The gain curve will obtain its maximum value of 6 dB when there is a relative delay between the two echoes equal to half the time period of the pulses corresponding to a delay of approximately 41.6 ns and 83.3 ns for the 12 MHz and 6 MHz pulses, shown as 301 and 302 respectively. It is for example seen in Figure 2 that for an imaging frequency of 12 MHz (301) and a delay equal to 4 ns, the detection signal is reduced by approximately 10 dB relative to a received bubble echo. When the imaging frequency is reduced to 6 MHz (302), a relative delay of approximately 8 ns will give a similar reduction of 10 dB in the detection signal relative to a received bubble echo.

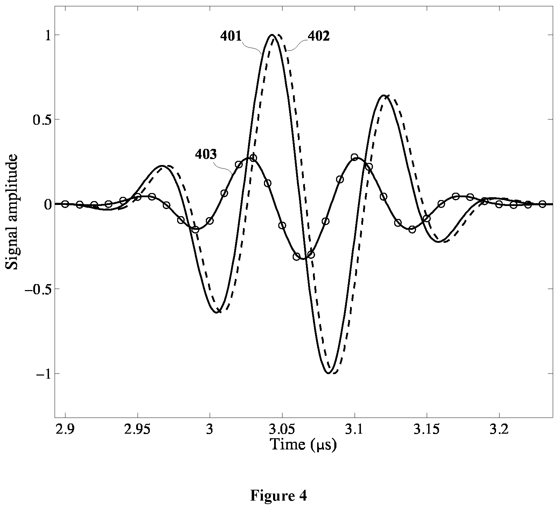

Figure 4 depicts a first signal 401 representing a first echo $x_1$ received from a given microbubble and a second signal 402 representing a second echo $x_2$ received from the same microbubble. The only difference between these two signals is a time delay of 5 ns and the two signals have a center frequency of 12 MHz. The curve 403 is obtained as the difference between 401 and 402 and represents the bubble detection signal $s$. Figure 3 illustrates how radiation forces from transmit pulses can induce a physical displacement with a corresponding time delay between echoes received from a given microbubble thus giving rise to a bubble detection signal.

A microbubble will, during interaction with the transmit pulses, typically undergo volumetric oscillations and possibly also surface oscillations at various surface modes. These oscillations will cause stresses in the shell encapsulating the gas core of the microbubble. During bubble oscillations, the mechanical properties of this encapsulating shell can potentially change (at least transiently) and a change in the mechanical properties of the shell will typically cause a change in the acoustic scattering properties of the microbubble. For example, the shell can have a thixotropic behavior where transmit pulses can manipulate and change (at least transiently) the acoustic scattering properties of the microbubble. It is also possible that one or several transmit pulses can cause a crack or other physical change in the shell of the microbubble that is small enough for the microbubble not to quickly disrupt and dissolve but large enough to cause a change in the acoustic scattering properties from the microbubble. A multi-pulse transmit scheme as suggested in the current invention is interesting for inducing such changes in the acoustic scattering properties from a microbubble, without causing rapid microbubble disruption, and hence for detection and imaging of the microbubble.

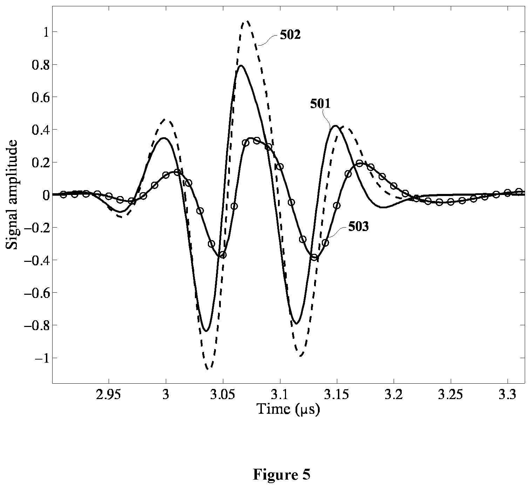

Figure 5 displays a first signal 501 representing a first echo $x_1$ received from a given microbubble and a second signal 502 representing a second echo $x_2$ received from the same microbubble. These two signals have a center frequency of 12 MHz and the curve 503 is obtained as the difference between 501 and 502 and represents the bubble detection signal $s$. The two signals 501 and 502 are different, the difference being introduced as a result of a change in the acoustic scattering properties of a given microbubble, for example due to a thixotropic behavior of the shell encapsulating the gas core of the microbubble. Figure 4 illustrates how transmit pulses can induce a change in the acoustic scattering properties from a given microbubble thus giving rise to a bubble detection signal.

To further increase the physical movement and/or the change in ultrasound scattering properties obtained for the microbubbles, it can be advantageous to apply more than two transmit pulses for each radial image line, for example as illustrated in Figure 1. One can then transmit a set of pulses along each radial image line where for the detection of microbubbles, a combination of the signals received from the transmitted imaging pulses will be used. The center frequency and bandwidth for the transmit pulses used for imaging are typically determined from a trade-off between spatial resolution and penetration of the object being imaged and will hence be determined based on the size and depth of the object being imaged. The intermediate transmit pulses can, however, have center frequencies and/or amplitudes and/or number of oscillations that are different from the transmit pulses used for image reconstruction. To increase the physical movement induced on a given microbubble, intermediate transmit pulses of long duration (consisting of several oscillations) are useful. The pulse repetition time for the transmitted imaging pulses is typically chosen to be a little longer than the time needed to receive the scattered signals from the deepest imaging depths. However, for the intermediate transmit pulses not used for image reconstruction, one does not have to wait for echoes to be received. The pulse repetition time for these intermediate transmit pulses is thus not limited by the time needed to receive echoes from a certain depth and can potentially be shorter than for the imaging transmit pulses.

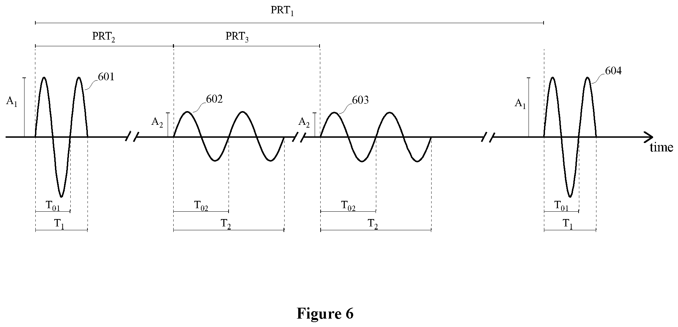

Figure 6 displays a first transmit pulse 601, a second transmit pulse 602, a third transmit pulse 603 and a fourth transmit pulse 604 that are all transmitted along the same radial image line. The first transmit pulse 601 and the last transmit pulse 604 have the same time period $T_{01}$, the same pulse duration $T_1$ and the same amplitude $A_1$ and the received echoes from these two imaging transmit pulses are used for image reconstruction. The intermediate transmit pulses 602 and 603 are used to enhance the physical movement and/or the change in ultrasound scattering properties for the contrast agent observed with the imaging transmit pulses. The intermediate transmit pulses can potentially have a time period $T_{02}$, pulse duration $T_2$ and amplitude $A_2$ that is different from the first and the last transmit pulses used for image reconstruction. In Figure 6, it is seen that when such a group of transmit pulses are used along a given radial image line, the pulse repetition time (PRT) can potentially vary between consecutive transmit pulses so that $PRT_1 \neq PRT_2 \neq PRT_3$.

In conventional focused ultrasound imaging, ultrasound waves are electronically focused on both transmit and receive along each radial image line. An ultrasound image is then made up from many (e.g. 128) radial image lines for the transmit pulses. In plane wave imaging, however, the transducer transmits an unfocussed wave resembling a plane wave and covering a large field of view. The echoes received from the transmitted unfocussed wave are collected on the transducer channels in parallel and beam-formed synthetically. To retrieve signal-to-noise ratio and contrast resolution it is common to transmit plane waves at various angles and compound the resulting beam-formed images. With a relatively small number of plane waves (typically around 10 or less), it is possible to obtain an image equivalent to conventional focused imaging but with a much higher frame rate. The embodiments of the invention described here for imaging of microbubbles are also interesting using plane wave imaging where unfocussed or weakly focused transmit pulses are then applied. One will in this situation have only one radial image line or a few radial image lines for the transmit pulses.

To suppress tissue artifacts from physiologic motion and transducer motion and hence to obtain adequate contrast agent specificity, it may in many imaging situations be advantageous to apply motion filters to compensate for and hence suppress the indicated tissue artifacts. Such motion filter can be based on algorithms driven by recorded data (received echoes), by model-based algorithms or by a combined approach using information from both recorded data and models.

## Claims

1. A method for detection and imaging of ultrasound contrast agent in a region, where:

   - at least two ultrasound pulses (601-604) are transmitted consecutively toward said region along a given radial image line,
   - at least one of said transmitted pulses induces a physical movement and/or at least transiently alters the acoustic scattering properties of the contrast agent,
   - the received signals (101, 104) from said at least two transmitted pulses are used as imaging signals to detect and image the contrast agent, and
   - the imaging signals are for each depth along said radial image line combined such that the signal components from the contrast agent are, at least to some degree, preserved for detection and imaging of the contrast agent while other signal components are suppressed; **characterized in that**:

     one or more of the transmitted pulses (601-604) has a mechanical index greater than 0.3; and/or
     the imaging frequency is at least three times higher than the resonance frequency of the contrast agent.

2. A method for detection and imaging of ultrasound contrast agent according to claim 1, wherein the region is a region of soft tissue and wherein the imaging signals (101, 104) are combined so as to suppress tissue signal components.

3. A method for detection and imaging of ultrasound contrast agent according to Claim 1 or 2, where:

   - the ultrasound transmit direction is scanned in steps, each step corresponding to a radial image line and for each radial image line a group of pulses (601-604) is transmitted,
   - the group of pulses comprises at least two imaging pulses (601, 604), each imaging pulse in the group having a pulse number,
   - the back-scattered signal is sampled as a function of depth for each imaging pulse and the samples for each depth are combined linearly along the pulse number coordinate,
   - the linear combination for each depth being selected so that the received signal components from the contrast agent are, at least to some extent preserved while other components are heavily suppressed, and
   - so that the output of the linear combination is used to form the image of the contrast agent for each depth along said radial image line.

4. A method for imaging and detection of ultrasound contrast agent according to Claim 1, 2 or 3, where in said transmitted pulses (601-604) the amplitude may be separately adjusted; and/or
   where in said transmitted pulses (601-604) the center frequency may be separately adjusted; and/or
   where in said transmitted pulses (601-604) the number of oscillations may be separately adjusted; and/or
   where between said transmitted pulses (601-604) the pulse repetition time may be separately adjusted.

5. A method as claimed in any preceding claim, wherein the transmitted ultrasound pulses comprise at least two imaging pulses (601, 604).

6. A method as claimed in claim 5, wherein said imaging pulses (601, 604) are identical.

7. A method as claimed in Claim 5 or 6, wherein one or more intermediate pulses (602, 603) are transmitted in between said at least two imaging pulses (601, 604).

8. A method as claimed in Claim 7, wherein one or more of the intermediate transmit pulses (602, 603) has a longer duration than the first transmit imaging pulse (601).

9. A method as claimed in any preceding claim, wherein one or more of the transmitted pulses (601-604) has a mechanical index greater than 0.5.

10. A method as claimed in any preceding claim, wherein the contrast agent comprises microbubbles with a shell having a thickness greater than 40 nm.

11. A method as claimed in any preceding claim, wherein the contrast agent comprises microbubbles and wherein one or more of the transmit pulses (601-604) has a frequency and/or number of oscillations and/or amplitude suitable for causing cracks or other physical changes in the shells of said microbubbles.

12. A method as claimed in any preceding claim, wherein the contrast agent is microbubbles and wherein the microbubbles are thixotropic.

13. A method as claimed in any preceding claim, wherein the transmitted pulses (601-604) are unfocussed and cover a large field of view, and

    - where various transmit angles can be used for the unfocussed transmitted imaging pulses, and
    - where the received signals (101, 104) from the unfocussed transmitted imaging pulses are collected on transducer channels in parallel, beam-formed synthetically and combined to suppress tissue signal components and thereby to image the ultrasound contrast agent.

14. A method as claimed in any preceding claim, where to suppress tissue signal components due to physiologic motion or transducer motion, motion filters driven by recorded data (received echoes), model-based algorithms or by a combined approach using information from both recorded data and models are applied.

15. An apparatus for imaging an ultrasound contrast agent, comprising:

    - a transmitter arranged to transmit pulses (601-604) comprising at least a first imaging pulse (601) and a second imaging pulse (604) along a given radial image line, wherein at least one of said pulses (601-604) transmitted prior to the second imaging pulse (604) is capable of inducing movement in the ultrasound contrast agent or is capable of altering the acoustic scattering properties of the ultrasound contrast agent,
    - a receiver arranged to receive echoes from the imaging pulses and generate imaging signals (101, 104),
    - a processor arranged to combine imaging signals to suppress tissue signal components for each depth along said radial image line and thereby to image the ultrasound contrast agent,
    **characterized in that**:

        one or more of the transmit pulses (601-604) has a mechanical index greater than 0.3; and/or
        the imaging frequency is at least three times higher than the resonance frequency of the contrast agent.

**Patentansprüche**

1. Verfahren zum Detektieren und Abbilden von Ultraschallkontrastmittel in einer Region, wobei:

    - mindestens zwei Ultraschallimpulse (601-604) nacheinander zu der Region hin entlang einer gegebenen radialen Bildzeile übertragen werden,
    - mindestens einer der übertragenen Impulse eine physische Bewegung induziert und/oder die akustischen Streuungseigenschaften des Kontrastmittels zumindest vorübergehend verändert,
    - die empfangenen Signale (101, 104) aus den mindestens zwei übertragenen Impulsen als Bildgebungssignale zum Detektieren und Abbilden des Kontrastmittels verwendet werden und
    - die Bildgebungssignale für jede Tiefe entlang der radialen Bildzeile so kombiniert werden, dass die Signalkomponenten aus dem Kontrastmittel, zumindest bis zu einem gewissen Grad, für das Detektieren und Abbilden des Kontrastmittels bewahrt werden, während andere Signalkomponenten unterdrückt werden; **dadurch gekennzeichnet, dass**
    einer oder mehrere der übertragenen Impulse (601-604) einen mechanischen Index von mehr als 0,3 aufweist;

und/oder

die Bildgebungsfrequenz mindestens drei Mal höher ist als die Resonanzfrequenz des Kontrastmittels.

2. Verfahren zum Detektieren und Abbilden von Ultraschallkontrastmittel nach Anspruch 1, wobei die Region eine Region aus weichem Gewebe ist und wobei die Bildgebungssignale (101, 104) so kombiniert werden, dass sie Gewebesignalkomponenten unterdrücken.

3. Verfahren zum Detektieren und Abbilden von Ultraschallkontrastmittel nach Anspruch 1 oder 2, wobei:

- die Ultraschallübertragungsrichtung schrittweise gescannt wird, wobei jeder Schritt einer radialen Bildzeile entspricht und für jede radiale Bildzeile eine Gruppe von Impulsen (601-604) übertragen wird,
- die Gruppe von Impulsen mindestens zwei Bildgebungsimpulse (601, 604) umfasst, wobei jeder Bildgebungsimpuls in der Gruppe eine Impulszahl aufweist,
- das zurückgestreute Signal als eine Funktion der Tiefe für jeden Bildgebungsimpuls abgetastet wird und die Abtastungen für jede Tiefe entlang der Impulszahlkoordinate linear kombiniert werden,
- die lineare Kombination für jede Tiefe so ausgewählt wird, dass die empfangenen Signalkomponenten aus dem Kontrastmittel, zumindest bis zu einem gewissen Grad, bewahrt werden, während andere Komponenten stark unterdrückt werden, und
- so dass die Ausgabe der linearen Kombination verwendet wird, um das Bild des Kontrastmittels für jede Tiefe entlang der radialen Bildzeile zu bilden.

4. Verfahren zum Abbilden und Detektieren von Ultraschallkontrastmittel nach Anspruch 1, 2 oder 3, wobei die Amplitude in den übertragenen Impulsen (601-604) separat eingestellt werden kann; und/oder

wobei die Mittenfrequenz in den übertragenen Impulsen (601-604) separat eingestellt werden kann; und oder

wobei die Anzahl an Oszillationen in den übertragenen Impulsen (601-604) separat eingestellt werden kann; und/oder

wobei die Impulsfolgezeit zwischen den übertragenen Impulsen (601-604) separat eingestellt werden kann.

5. Verfahren nach einem der vorstehenden Ansprüche, wobei die übertragenen Ultraschallimpulse mindestens zwei Bildgebungsimpulse (601, 604) umfassen.

6. Verfahren nach Anspruch 5, wobei die Bildgebungsimpulse (601, 604) identisch sind.

7. Verfahren nach Anspruch 5 oder 6, wobei ein oder mehrere Zwischenimpulse (602, 603) zwischen den mindestens zwei Bildgebungsimpulsen (601, 604) übertragen werden.

8. Verfahren nach Anspruch 7, wobei einer oder mehrere der Zwischensendeimpulse (602, 603) eine längere Dauer aufweist als der erste Bildgebungssendeimpuls (601).

9. Verfahren nach einem der vorstehenden Ansprüche, wobei einer oder mehrere der übertragenen Impulse (601-604) einen mechanischen Index von mehr als 0,5 aufweist.

10. Verfahren nach einem der vorstehenden Ansprüche, wobei das Kontrastmittel Mikroblasen mit einer Hülle in einer Dicke von mehr als 40 nm umfasst.

11. Verfahren nach einem der vorstehenden Ansprüche, wobei das Kontrastmittel Mikroblasen umfasst und wobei einer oder mehrere der Sendeimpulse (601-604) eine Frequenz und/oder Anzahl an Oszillationen und/oder Amplitude aufweist, die geeignet sind, Risse oder andere physische Veränderungen in den Hüllen der Mikroblasen zu bewirken.

12. Verfahren nach einem der vorstehenden Ansprüche, wobei das Kontrastmittel Mikroblasen umfasst und wobei die Mikroblasen thixotrop sind.

13. Verfahren nach einem der vorstehenden Ansprüche, wobei die übertragenen Impulse (601-604) nicht fokussiert sind und ein großes Sichtfeld abdecken und

- wobei verschiedene Sendewinkel für die nicht fokussierten übertragenen Bildgebungsimpulse verwendet werden können und
- wobei die empfangenen Signale (101, 104) aus den nicht fokussierten übertragenen Bildgebungsimpulsen parallel auf Transducer-Kanälen gesammelt, synthetisch zu einem Strahl geformt und kombiniert werden, um Gewebesignalkomponenten zu unterdrücken und dadurch das Ultraschallkontrastmittel abzubilden.

14. Verfahren nach einem der vorstehenden Ansprüche, wobei zur Unterdrückung von Gewebesignalkomponenten infolge von physiologischer Bewegung oder Transducer-Bewegung Bewegungsfilter angewendet werden, die von aufgezeichneten Daten (empfangenen Echos), Modell-basierten Algorithmen oder einem kombinierten Ansatz unter Verwendung von Informationen sowohl aus aufgezeichneten Daten als auch aus Modellen getrieben werden.

15. Vorrichtung zum Abbilden eines Ultraschallkontrastmittels, umfassend:

- einen Transmitter, der angeordnet ist, Impulse (601-604), die mindestens einen ersten Bildgebungsimpuls (601) und einen zweiten Bildgebungsimpuls (604) umfassen, entlang einer gegebenen radialen Bildzeile zu übertragen, wobei mindestens einer der Impulse (601-604), die vor dem zweiten Bildgebungsimpuls (604) übertragen werden, in der Lage ist, Bewegung in dem Ultraschallkontrastmittel zu induzieren, oder in der Lage ist, die akustischen Streuungseigenschaften des Ultraschallkontrastmittels zu verändern,
- einen Empfänger, der angeordnet ist, Echos aus den Bildgebungsimpulsen zu empfangen und Bildgebungssignale (101, 104) zu erzeugen,
- einen Prozessor, der angeordnet ist, Bildgebungssignale zu kombinieren, um Gewebesignalkomponenten für jede Tiefe entlang der radialen Bildzeile zu unterdrücken und dadurch das Ultraschallkontrastmittel abzubilden, **dadurch gekennzeichnet, dass**:

einer oder mehrere der übertragenen Impulse (601-604) einen mechanischen Index von mehr als 0,3 aufweist; und/oder
die Bildgebungsfrequenz mindestens drei Mal höher ist als die Resonanzfrequenz des Kontrastmittels.

**Revendications**

1. Procédé de détection et d'imagerie d'un agent de contraste ultrasonore dans une région, dans lequel :

- au moins deux impulsions ultrasonores (601-604) sont transmises de manière consécutive vers ladite région le long d'une ligne d'image radiale donnée,
- au moins l'une desdites impulsions transmises induit un mouvement physique et/ou au moins modifie momentanément les propriétés de diffusion acoustique de l'agent de contraste,
- les signaux reçus (101, 104) provenant desdites au moins deux impulsions transmises sont utilisés comme signaux d'imagerie pour détecter et imager l'agent de contraste, et
- les signaux d'imagerie sont, pour chaque profondeur le long de ladite ligne d'image radiale, combinés de sorte que les composantes des signaux venant de l'agent de contraste soient, au moins à un certain degré, préservées pour la détection et l'imagerie de l'agent de contraste tandis que d'autres composantes des signaux sont supprimées ; **caractérisé en ce que** :

une ou plusieurs des impulsions transmises (601-604) a un indice mécanique supérieur à 0,3 ; et/ou
la fréquence d'imagerie est au moins trois fois supérieure à la fréquence de résonance de l'agent de contraste.

2. Procédé de détection et d'imagerie d'un agent de contraste ultrasonore selon la revendication 1, dans lequel la région est une région de tissu mou et dans lequel les signaux d'imagerie (101, 104) sont combinés de manière à supprimer les composantes des signaux du tissu.

3. Procédé de détection et d'imagerie d'un agent de contraste ultrasonore selon la revendication 1 ou 2, dans lequel :

- la direction de transmission des ultrasons est balayée par étapes, chaque étape correspondant à une ligne d'image radiale et, pour chaque ligne d'image radiale, un groupe d'impulsions (601-604) est transmis,
- le groupe d'impulsions comprend au moins deux impulsions d'imagerie (601, 604), chaque impulsion d'imagerie du groupe ayant un nombre d'impulsions,
- le signal rétrodiffusé est échantillonné en fonction de la profondeur pour chaque impulsion d'imagerie et les échantillons pour chaque profondeur sont combinés de manière linéaire le long de la coordonnée du nombre d'impulsions,
- la combinaison linéaire pour chaque profondeur étant sélectionnée de sorte que les composantes de signaux

EP 3 125 770 B1

reçues de l'agent de contraste soient, au moins dans une certaine mesure, préservées tandis que d'autres composantes sont fortement supprimées, et

- de sorte que la sortie de la combinaison linéaire soit utilisée pour former l'image de l'agent de contraste pour chaque profondeur le long de ladite ligne d'image radiale.

4. Procédé d'imagerie et de détection d'un agent de contraste ultrasonore selon la revendication 1, 2 ou 3, dans lequel, dans lesdites impulsions transmises (601-604), l'amplitude peut être ajustée séparément ; et/ou

dans lequel, dans lesdites impulsions transmises (601-604), la fréquence centrale peut être ajustée séparément ; et/ou

dans lequel, dans lesdites impulsions transmises (601-604), le nombre d'oscillations peut être ajusté séparément ; et/ou

dans lequel, entre lesdites impulsions transmises (601-604), le temps de répétition des impulsions peut être ajusté séparément.

5. Procédé selon l'une quelconque des revendications précédentes, dans lequel les impulsions ultrasonores transmises comprennent au moins deux impulsions d'imagerie (601, 604).

6. Procédé selon la revendication 5, dans lequel lesdites impulsions d'imagerie (601, 604) sont identiques.

7. Procédé selon la revendication 5 ou 6, dans lequel une ou plusieurs impulsions intermédiaires (602, 603) est ou sont transmises entre lesdites au moins deux impulsions d'imagerie (601, 604).

8. Procédé selon la revendication 7, dans lequel une ou plusieurs des impulsions de transmission intermédiaires (602, 603) a ou ont une durée plus longue que la première impulsion d'imagerie de transmission (601).

9. Procédé selon l'une quelconque des revendications précédentes, dans lequel une ou plusieurs des impulsions transmises (601-604) a ou ont un indice mécanique supérieur à 0,5.

10. Procédé selon l'une quelconque des revendications précédentes, dans lequel l'agent de contraste comprend des microbulles ayant une coque d'une épaisseur supérieure à 40 nm.

11. Procédé selon l'une quelconque des revendications précédentes, dans lequel l'agent de contraste comprend des microbulles et dans lequel une ou plusieurs des impulsions de transmission (601-604) a ou ont une fréquence et/ou un nombre d'oscillations et/ou une amplitude convenant pour provoquer des fissures ou d'autres changements physiques dans les coques desdites microbulles.

12. Procédé selon l'une quelconque des revendications précédentes, dans lequel l'agent de contraste est formé de microbulles et dans lequel les microbulles sont thixotropes.

13. Procédé selon l'une quelconque des revendications précédentes, dans lequel les impulsions transmises (601-604) ne sont pas focalisées et couvrent un grand champ de vision, et

- dans lequel divers angles de transmission peuvent être utilisés pour les impulsions d'imagerie transmises non focalisées, et
- dans lequel les signaux reçus (101, 104) venant des impulsions d'imagerie transmises non focalisées sont recueillis sur des canaux de transducteur en parallèle, conformés en faisceau par synthèse et combinés pour supprimer les composantes de signaux du tissu et ainsi imager l'agent de contraste ultrasonore.

14. Procédé selon l'une quelconque des revendications précédentes, dans lequel, pour supprimer les composantes de signaux de tissu dues à un mouvement physiologique ou à un mouvement de transducteur, des filtres en mouvement entraînés par des données enregistrées (échos reçus), des algorithmes à base de modèle ou par une approche combinée en utilisant des informations venant à la fois des données enregistrées et des modèles sont appliqués.

15. Appareil d'imagerie d'un agent de contraste ultrasonore, comprenant :

- un émetteur agencé pour transmettre des impulsions (601-604) comprenant au moins une première impulsion d'imagerie (601) et une seconde impulsion d'imagerie (604) le long d'une ligne d'image radiale donnée, dans lequel au moins l'une desdites impulsions (601-604) transmise avant la seconde impulsion d'imagerie (604) est capable d'induire un mouvement dans l'agent de contraste ultrasonore ou est capable de modifier les propriétés de diffusion acoustique de l'agent de contraste ultrasonore,

- un récepteur agencé pour recevoir des échos des impulsions d'imagerie et générer des signaux d'imagerie (101, 104),

- un processeur agencé pour combiner des signaux d'imagerie afin de supprimer des composantes de signaux de tissu pour chaque profondeur le long de ladite ligne d'image radiale et ainsi imager l'agent de contraste ultrasonore,

**caractérisé en ce que** :

une ou plusieurs des impulsions de transmission (601-604) a ou ont un indice mécanique supérieur à 0,3 ; et/ou

la fréquence d'imagerie est au moins trois fois supérieure à la fréquence de résonance de l'agent de contraste.

Figure 1

**Figure 2**

**Figure 3**

**Figure 4**

**Figure 5**

**Figure 6**

**EP 3 125 770 B1**

**REFERENCES CITED IN THE DESCRIPTION**

**Patent documents cited in the description**

- US 20020028994 A
- US 20040236222 A
- US 20040267129 A